# EUROPEAN PATENT SPECIFICATION

(11) **EP 1 809 224 B1**
(45) Date of publication and mention of the grant of the patent: **07.11.2012**
(21) Application number: 05800730.3
(22) Date of filing: 26.09.2005
(51) Int. Cl.: A61F 13/496, A61F 13/62, A61F 13/58, A61F 13/15, A61F 13/56

(54) **RE-ATTACHABLE ABSORBENT ARTICLE AND METHOD OF MAKING THE SAME**
WIEDER BEFESTIGBARER SAUGFÄHIGER ARTIKEL UND HERSTELLUNGSVERFAHREN DAFÜR
ARTICLE ABSORBANT FIXABLE PLUSIEURS FOIS ET SON PROCEDE DE FABRICATION

(30) Priority: 28.09.2004 US 613837 P
(43) Date of publication of application: 25.07.2007
(73) Proprietor: Tyco Healthcare Retail Services AG, 8212 Neuhausen am Rheinfall (CH)
(72) Inventor: GLAUG, Frank, S., Chester Springs, PA 19425 (US)
(74) Representative: Elsy, David
(86) International application number: PCT/US2005/034288
(87) International publication number: WO 2006/036841

(56) References cited:
- GB-A- 2 315 402
- US-A- 5 549 592
- US-A1- 2002 138 064
- US-A1- 2003 158 532
- US-A1- 2003 167 049

## Description

### TECHNICAL FIELD

The present invention relates generally to absorbent articles and, more particularly, to absorbent under-garments such as diapers, training pants, adult incontinence garments, and the like.

### BACKGROUND OF THE INVENTION

Absorbent articles such as disposable diapers, training pants, adult incontinence garments, and the like are known in the art. In the past, particularly in the case of infant diapers, such absorbent articles were generally formed with an hourglass configu ration. The narrower portion of the article was adapted to be placed between the legs of the wearer with the wider portions of the article being adapted to encircle the waist of a wearer so that the front and rear portions overlapped and could be easily attached to one another. Recently, it has become desirable to produce absorbent articles, such as infant diapers, which fit more closely to the body of a wearer. Accordingly, it has become desirable to make such articles smaller and less conspicuous in use while still maintaining a high level of absorbent protection. Specifically, it has become desirable to produce disposable absorbent articles which have a relatively narrow crotch section and a narrower overall width when compared to typical disposable absorbent articles.

Diapers and other such absorbent garments having an hourglass configuration are generally formed by cutting out or otherwise removing material in order to form leg cutouts. The cut out material is wasted, introducing undesirable manufacturing costs. Alternatively, an hourglass configuration can be formed by applying an elastic material to a chassis of the absorbent garment, thereby forming panels that can be overlapped and attached to one another. Such elastic panels may, however, introduce undesirable manufacturing costs. Furthermore, the elastic materials used to form such panels may suffer from a lack of breathability.

Accordingly, it is desirable to provide an improved absorbent article which corrects for the perceived deficiencies and undesirable aspects of known absorbent articles.

### SUMMARY OF THE INVENTION

According to one aspect of the invention, an absorbent article comprising an absorbent body having a substantially rectangular perimeter, a front portion configured to be positioned against a front of a wearer, and a back portion configured to be positioned against a back of a wearer is provided. At least one panel is attached to the back portion of the absorbent body and extends outwardly from the perimeter of the absorbent body. The panel may be formed from one or more non-woven, non-elastic materials. An elastic member is attached to and extends outwardly from the panel and a fastener is coupled to the elastic member for engaging the front portion of the absorbent body.

According to another aspect of the invention, a method for producing an absorbent article is provided. The method comprises the step of attaching at least one panel formed of a non-woven, non-elastic material to a back portion of a substantially rectangular absorbent body such that the panel extends outwardly from a perimeter of the absorbent body. An elastic member is attached to the panel such that the elastic member extends outwardly from the panel. A fastener is coupled to the elastic member, wherein the fastener is configured to engage the front portion of the absorbent body.

According to yet another aspect of the invention, a method for producing an absorbent article in a pre-attached state is provided. The method comprises the step of coupling a fastener to the elastic member and engaging the fastener with a front portion of the absorbent body to form a pre-attached absorbent article.

It is to be understood that both the foregoing general description and the following detailed description are exemplary, but are not restrictive, of the invention.

### BRIEF DESCRIPTION OF THE DRAWING

The invention is best understood from the following detailed description when read in connection with the accompanying drawing, which shows exemplary embodiments of the invention selected for illustration. It is emphasized that, according to common practice, the various features of the drawing are not to scale. On the contrary, the dimensions of the various features are arbitrarily expanded or reduced for clarity. Included in the drawing are the following figures:
Fig. 1 is a perspective drawing of an exemplary embodiment of an absorbent garment, according to the present invention;
Fig. 2A is a top plan drawing of an exemplary embodiment of two non-woven panels, according to the present invention;
Fig. 2B is a top plan drawing of another exemplary embodiment of two non-woven panels, according to the present invention;
Fig. 2C is a top plan drawing of an exemplary embodiment of a non-woven panel, according to the present invention;
Fig. 3A is a top schematic drawing of another embodiment of an absorbent garment shown unfastened, according to the present invention;
Fig. 3B is a front schematic drawing of the absorbent garment of Fig. 3A shown fastened, according to the present invention;
Fig. 4 is a cross-sectional drawing of an embodiment of an absorbent garment taken along the lines III-III in Fig. 3A, according to the present invention;
Fig. 5A is a top schematic drawing of another embodiment of an absorbent garment shown unfastened, according to the present invention;
Fig. 5B is a front schematic drawing of the absorbent garment of Fig. 5A shown fastened, according to the present invention; and
Fig. 6 is a flow-chart diagram illustrating an exemplary embodiment of a method of manufacturing an absorbent garment, according to the present invention.

### DETAILED DESCRIPTION OF THE INVENTION

Exemplary embodiments of the invention will now be described with reference to the drawing. It will be appreciated that the invention is not limited to the embodiments selected for illustration in the drawing. Various modifications can be made to the illustrated and described embodiments within the spirit and scope of this invention.

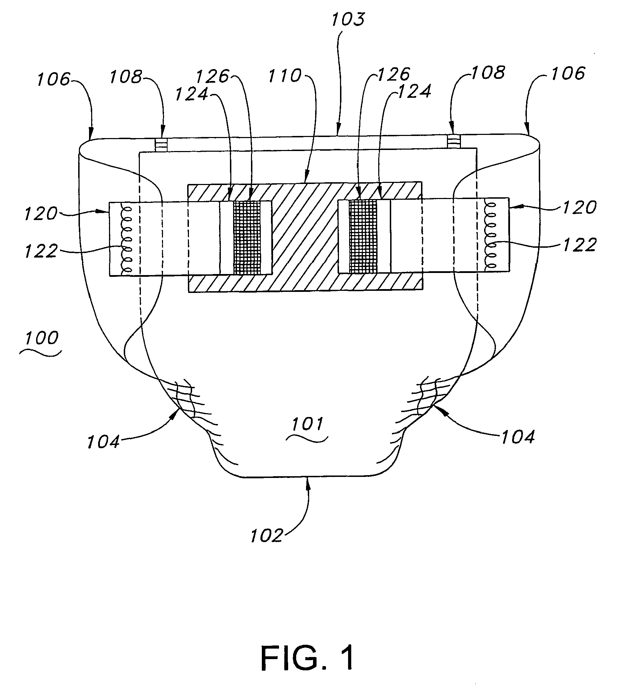

Referring generally to the drawings, (Figures 1, 3A, 3B, 5A, 5B) an absorbent article 100, 200, 400 includes an absorbent body 102, 202, 402 having a perimeter, a front portion 101, 201, 401 and a back portion 103, 203, 403. At least one panel 106, 206, 406 is attached to the back portion 103, 203, 403 of the absorbent body 102, 202, 402 and extends outwardly from the perimeter of the absorbent body 102, 202, 402. The panel 106, 206, 406 is formed from a single or multiple layers of non-woven(s), non-elastic material. An elastic member 120, 220, 420 is attached to and extends outwardly from the panel 106, 206, 406, and a fastener 124, 224, 424 is coupled to the elastic member 120, 220, 420 for engaging the front portion 101, 201, 401 of the absorbent body 102, 202, 402.

In a further embodiment of the present invention, a method for producing an absorbent article 100, 200, 400 includes attaching a panel 106, 206, 406 formed from a single or multiple layers of non-woven(s), non-elastic material to a back portions 103, 203, 403 of an absorbent body 102, 202, 402 such that the panel 106, 206, 406 extends outwardly from a perimeter or the absorbent body 102, 202, 402. An elastic member 120, 220, 420 is attached to the panel 106, 206, 406 such that the elastic member 120, 220, 420 extends outwardly from the panel 106, 206, 406, and a fastener 124, 224, 424 is coupled to the elastic member 120, 220, 420 for engaging the front portion 101, 201, 401 of the absorbent body 102, 202, 402.

In one exemplary embodiment of the present Intention, an absorbent garment is provided as having an absorbent chassis, two inelastic non-woven side panel components bonded to the back of the chassis, a hook or loop region formed on the front of the chassis, and two rectangular elastic members having fasteners that may be attached to the hook or loop region, where the two rectangular elastic members may be bonded to a respective panel component.

Referring now to the drawing, in which like reference numbers refer to like elements throughout the various figures that comprise the drawing, Fig. 1 is a perspective drawing of an absorbent article or garment 100 according to one embodiment of the present invention. As shown in the figure, absorbent garment 100 is comprised of an absorbent/garment body (or chassis) 102 having a front 101 and a back 103. Absorbent body 102 may be further comprised of elastic leg gathers 104 for allowing a secure fit between the absorbent garment and a user wearing the absorbent garment. Furthermore, absorbent body 102 may be formed from a plurality of layers of material, some of which may be absorbent, and may generally be an absorbent assembly known by those skilled in the art.

Attached to each of the right and left sides of the back 103 of garment body 102 is an individual panel component (or ear) 106, which may be attached by a bond 108. The panel components extend outwardly from a perimeter of the absorbent body 102. Bond 108 may be an adhesive bond, an ultrasonic bond, a heat seal, or a combination thereof or any equivalent fastening mechanism known in the art. Panel component 106 is formed from a non-woven material, and exhibits little to no elastic/stretch properties (i.e., inelastic). It may be formed of a single layer. Further, panel component 106 may be formed of a different material than absorbent body 102, thereby desirably allowing the use of less expensive, more breathable, and lower stiffness materials for panel components 106 than absorbent body 102.

The panel component (or ear) 106 is optionally forced from a laminated sheet of a non-woven material and film (with the non-woven side positioned as the outermost layer). One particularly suitable material is a spunbond-meltblow-spunbond (SMS) web having a basis weight of about 10 to 20 gms per square meter (gsm), available from AVGOL Nonwoven Industries LTD., Holon, Israel. The spunbond layer is optionally made of polypropylene fibers. Such composites provide the dual advantages of liquid barrier properties of film along with a soft, warm outer fabric texture. The panel component (or ear) 106 can also be made or other suitable cloth-like materials, e.g., spun-bond or thermal-bond non-woven web made of either polypropylene, polyethylene, polyester, bi-component fibers (polyethylene/polypropylene or polyethylene/polyester), or any combinations of these fibers. Various multiple layer configurations or fiber denier variations may be used. Another example includes hydroentangled non-woven webs, which may contain some cotton and/or rayon fibers blended in with thermal-plastic timbers. Cellulose fibers can also be blended in at small percentage to reduce cost. Still another example of an exemplary material is stretchable or elastic materials, such as elastomeric composites of non-woven(s) and elastic membranes or a single layer of elastic material. The elastomeric composite can comprise an inner layer of pre-stretched extruded elastic film sandwiched between and attached to a pair of non-woven webs. The non-woven webs may consist of spun-bond web, thermal-bond web, or a combination of the two. Optionally, the elastic film is made of synthetic rubber and the non-woven made of spun-bond polypropylene.

Still another example is a film made of a "breathable microporous polyethylene. Exemplary breathable films are available from Tredegar Film Products, Richmond, VA. This material allows water vapor to pass through it over time, while being impervious to liquid water. The water vapour transmission rate may range from 200-2000 grams per square meter per 24 hour period. Other materials for forming the breathable panel component 106 may include other breathable films, such as polypropylene films, co-extruded films (polyethylene and ethylene vinyl acetate), co-polymer films (polyethylene/polypropylene), and polylaminates (polypropylene nonwoven and polyethylene film).

The panel component 106 is optionally selected from a variety of textile-like films and fabrics. Suitable fabrics include non-woven materials that are impervious to liquid, soft and pliable. Exemplary non-woven materials include spun-bonded polypropylene, spunbonded polyethylene, and thermally bonded webs of staple fibers, preferably sheath/core bi-component fibers having a core of polyester or polypropylene and a sheath of polyethylene.

Elastic members (or tabs) 120 are attached to the ends of panel component 106, opposite the bonds 108. The elastic members (or tabs) 120 may be attached to a respective non-woven panel component 106 with bond 122. In one embodiment of the present invention, elastic members 120 may substantially be a rectangle or parallelogram, and it may be desirable to have the width of elastic member 120 be smaller than the adjacent width of panel component 106, but such width can be the same or greater. Further, bond 122 may be an adhesive bond, an ultrasonic bond, a heat seal, or a combination thereof or any other known fastening mechanism. The elastic members may be formed from a fluted elastic or stretch non-woven laminate material obtained, for example, from Tredegar Film Products of Richmond, VA. Other elastic and non-elastic materials may be used as well, depending on design considerations.

Additionally, each elastic member 120 may include a non-permanent reattachment means 124 on the opposite end from the permanent bond 122 to panel component 106. Reattachment means 124 may include a hook assembly 126 that may be non-permanently attached to loop 110 on front 101 of absorbent body 102. Alternatively, a fastening tape 126 may be non-permanently attached to frontal tape 110 on front 101 of absorbent body 102. Reattachment means 124 may be provided by any known releasable fastener, including adhesives, tapes, buttons, and other fastening mechanisms used in the art. Elastic member 120 may further include a supplemental tab component or portion (not shown) to allow easy detachment of hook (or fastening tape) assembly 126 from loop (or frontal tape) 110, or, in general, reattachment means 124 from absorbent body 102.

In one embodiment of the invention, elastic member 120 may be formed from a high-stretch laminate comprising an elastic middle layer sandwiched in between two cloth-like materials. Reattachment means 124, furthermore, may comprise an inelastic material and may further include a finger tab at one end, or disposed along its perimeter.

According to one embodiment of the present invention, elastic members 120 may be already attached (pre-attached) to absorbent body 102 through the coupling of hook fastener assembly 126 (or fastening tape) and loop fastener 110 (or frontal tape), as part of the manufacturing process (i.e., the absorbent garment may come pre-assembled and ready to be worn by a user). Although the garment body 102 utilizes VELCRO-type fasteners, the garment body may employ any fastening means known in the art. Alternately, the garment may be manufactured and shipped unattached, thereby requiring a user to fold front 101 and back 103 of garment 100 adjacent one another and attach elastic member 120 to absorbent body 102 by coupling hook fastener assembly 126 (or fastening tape) to loop fastener 110 (or frontal tape) or requiring a user to don the garment 100 like a conventional absorbent article.

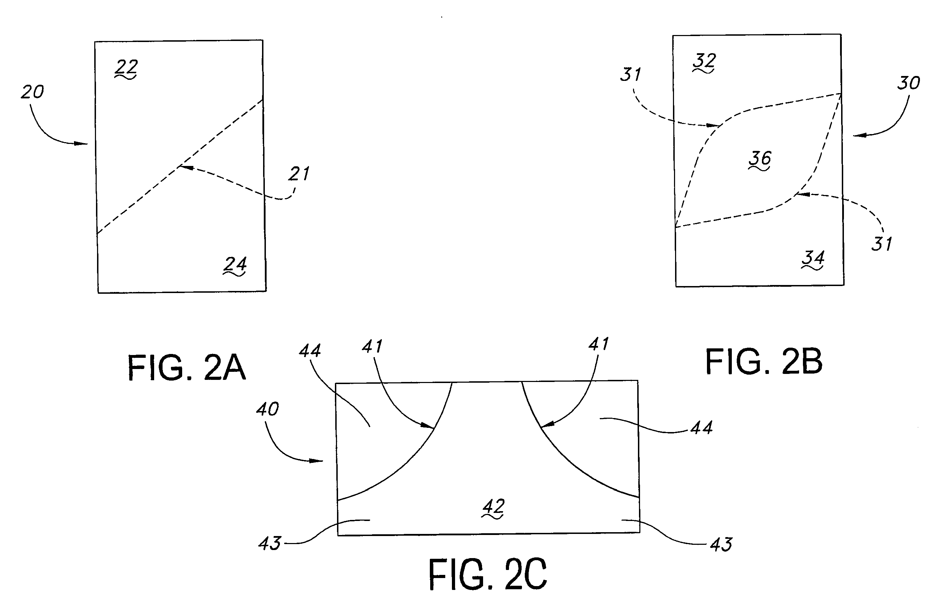

Figs. 2A-C illustrate various exemplary embodiments of panel component 106, in a pre-assembled form. In the embodiments shown in Figs. 2A-C, the panel component (or ear) material is represented by the numerals 20, 30 and 40, respectively. In Fig. 2A, a piece of single layered, non-woven, inelastic material 20, for example, is cut along cut line 21 to form the two panel (or ear) components 22 and 24 configured for use on the absorbent garment, as described above. The panel components 22 and 24 may represent a panel component similar to panel 106 illustrated in Fig. 1. As can be seen, the embodiment shown in Fig. 2A wastes little panel component material 20.

In some embodiments, however, it may be desirable to provide panel components with some amount of curvature. Accordingly, Fig. 2B shows that inelastic material 30 may be cut along cut lines 31 to form the two panel components 32 and 34. The curvature of cut lines 31, therefore, may result in waste material 36 being wasted in the manufacturing of the panel components.

In further embodiments, it may be desirable to provide the panel component as a single uninterrupted piece of material. Accordingly, Fig. 2C illustrates a panel component 42 cut from inelastic material 40 along cut lines 41. The remaining material 44 is waste material. The ends 43 of the panel component 42 are adapted to extend beyond a perimeter of an absorbent body. In such an embodiment, those skilled in the art will recognize that bond 108 may be desirably disposed around the perimeter of panel component 42 that is in direct contact with the absorbent body (not shown in Fig. 2C).

Those skilled in the art will recognize that other geometries of the panel component may be implemented and attached to the absorbent body using other types of adhesives or fastening without departing from the present invention.

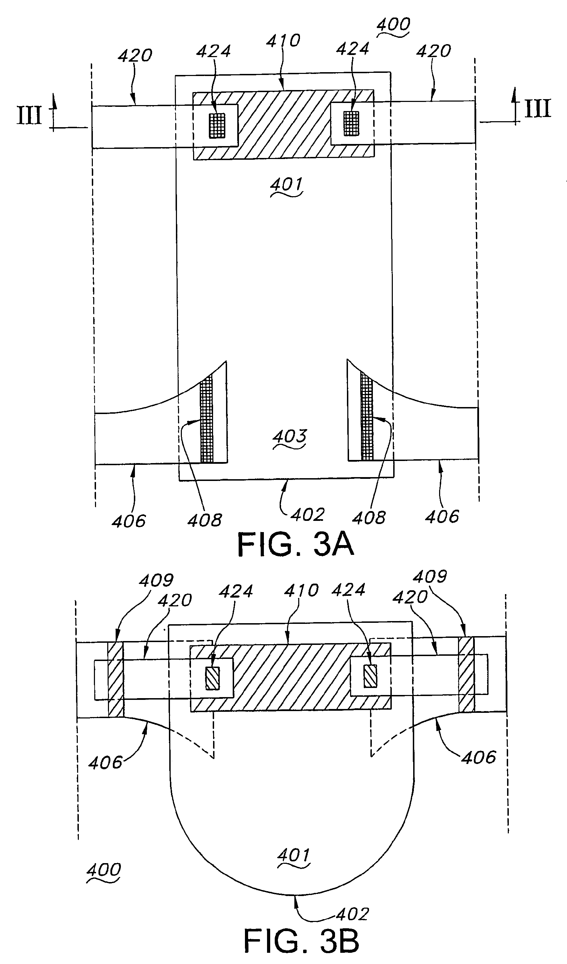

Referring now to Fig. 3A, a top plan view is shown of an exemplary absorbent garment 400 according to another aspect of the present invention. Garment 400 includes a rectangular absorbent body 402 having a front 401 and a back 403. Attached to back 403 are two non-woven ears 406 that may be permanently attached with bonds 408, which may be adhesive bonds, ultrasonic bonds, heat seals, or combinations thereof, for example. Attached to front 401 are two elastic tabs 420 that may be non-permanently attached with reattachment means 424 to a landing zone such as loop 410 of absorbent body 402. Reattachment means 424 may be a hook assembly that couples to loop 410. Alternatively, the reattachment means may include an adhesive bond or other known fastening system.

Fig. 3A shows elastic tabs 420 as being separate from non-woven ears 406 only for illustrative purposes. In actuality, non-woven ears 406 may be bonded or otherwise attached to (or integral with) elastic tabs 420, as described above. The broken lines in Fig. 3A Illustrate the relationship between the tabs 420 and ears 406. Specifically, those lines show one possible border between the panels and tabs.

Fig. 3B shows the absorbent garment of Fig. 3A in the folded position with non-woven ears 406 attached to elastic tabs 420 with bonds 409. Bonds 409 may be adhesive bonds, ultrasonic bonds, heat seals, or combinations thereof, or any other known fastening system.

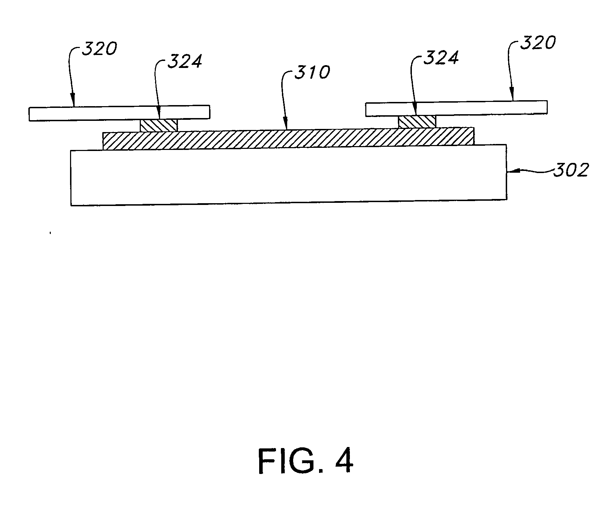

Fig. 4 is a cross-sectional view of the front of an absorbent body of one ern bodiment of the present invention, such as that of Fig. 3A, taken along cut-away line III. Accordingly, an absorbent body 302 is shown which may be any absorbent body known by those skilled In the art. Loop 310 or an alternative fastener may be coupled to the top of absorbent body 302, or may alternately be included as the top layer of absorbent body 302. Elastic member 320 having hooks 324 or another suitable fastener may then be non-permanently coupled to absorbent body 302 by placing hooks 324 at desirable locations along loop 310.

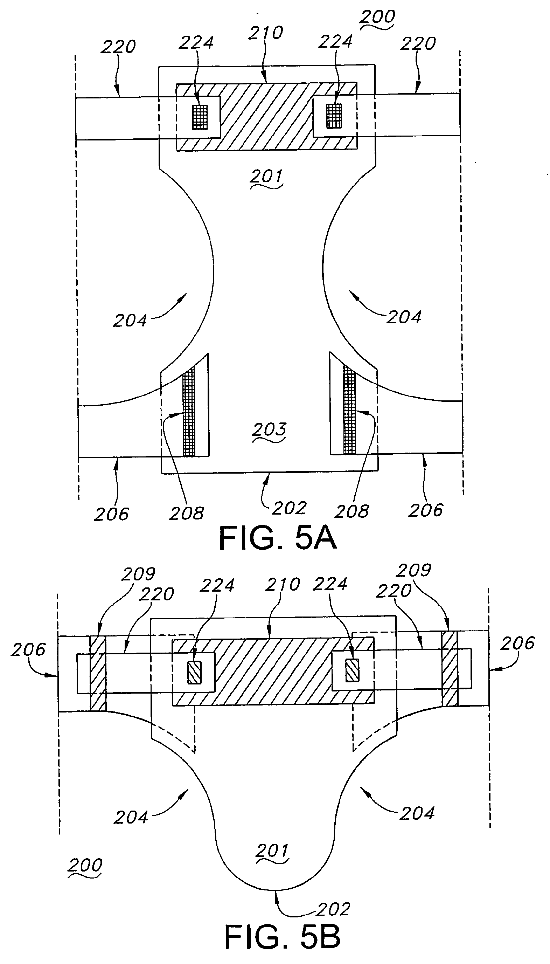

Referring now to Fig. 5A, a top plan view is shown of a further exemplary absorbent garment 200 according to the present Invention. Garment 200 includes a shipped absorbent body 202 having a front 201 and a back 203, and further including leg cut-outs 204, where material from absorbent body 202 has been removed.

Attached to back 203 are two non-woven ears 206 that may be permanently attached with bonds 208, which may be adhesive bonds, ultrasonic bonds, heat seals, or combinations thereof, for example. Attached to front 201 are two elastic tabs 220 that may be non-permanently attached with reattachment means 224 coupled bo loop 210 of absorbent body 202. Reattachment means 224 may be a hook assembly that couples to loop 210. Alternately, the reattachment means may include an adhesive bond or other suitable fastening mechanism known in the art.

Fig 5A shows elastic tabs 220 as being separate from non-woven ears 206 only for illustrative purposes. In actuality, non-woven ears 206 are bonded or otherwise attached to (or integral with) elastic tabs 220, as described above. The broken lines in Fig. 5A illustrate the relationship between the tabs 220 and cars 206. Specifically, those lines show one possible border between the panels and tabs.

Fig. 5B shows the absorbent garment of Fig. 5A in the folded position with non-woven ears 206 attached to elastic tabs 220 with bonds 209. Bonds 209 may be adhesive bonds, ultrasonic bonds, heat seals, or combinations thereof, or any other suitable coupling system.

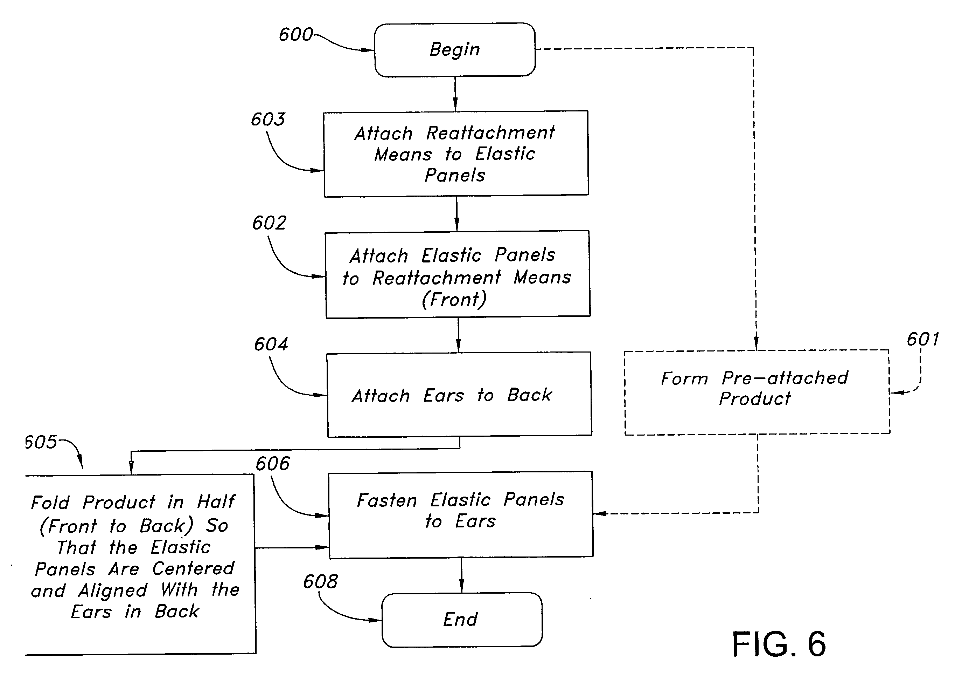

According to a further embodiment of the invention, Fig. 6 is a flow-chart diagram illustrating a method of manufacture of an absorbent article (or garment). The method begins at step 600 and proceeds to step 603, where reattachment means are attached to one or more elastic members, and then to step 602, where one or more elastic members (or tabs) are attached to a reattachment means on the product, such that each elastic member extends outwardly from the respective panel. The method then proceeds to step 604, where one or more of the panels are attached to the back portion of a substantially rectangular absorbent body such that the one or more panels extend outwardly from a respective perimeter of the absorbent body. Alternately, step 604 may be performed prior to step 602. Furthermore, the one or more panels may have been previously formed from a single layer of non-woven, non-elastic material.

Step 605 involves folding the product in half, aligning the front end-cut to the back end-cut, and centering the Elastic Panels (Front) to the Ears (Back) respectively. Step 606 proceeds by fastening or bonding the Elastic Panel (Front) to the Ears (back), extending outwardly from the perimeter of the absorbent body. Step 601 illustrates the process for manufacturing the product in a pre-attached state. The method ends in step 608.

Although shown as being performed sequentially, the steps in the above method may be performed in any order, or, alternately, in parallel. Also, exemplary steps illustrated in Fig. 6 may be omitted depending upon the design selected for the absorbent garment, manufacturing efficiencies, cost considerations and other factors.

Although illustrated and described above with reference to certain specific embodiments, the present invention is nevertheless not intended to be limited to the details shown. Rather, various modifications may be made within the scope and range of equivalents of the claims and without departing from the invention.

## Claims

1. An absorbent article (100; 400) comprising:
an absorbent body (102; 402) having a substantially rectangular perimeter, a front portion (101; 401) configured to be positioned against a front of a wearer, and a back portion (103; 403) configured to be positioned against a back of a wearer;
at least one panel (106; 406) attached to said back portion of said absorbent body and extending outwardly from said perimeter of said absorbent body, said panel being formed from one or more non-woven materials;
**characterized in that** said absorbent article further comprises an elastic member (120; 420) attached to and extending outwardly from said at least one panel, and **in that** said non-woven material is non-elastic and breathable; and
a fastener (124, 126; 424) coupled to said elastic member for engaging said front portion of said absorbent body.

2. The absorbent article of claim 1 wherein said absorbent article includes two panels (106; 406) attached to said back portion of said absorbent body and extending outwardly from said perimeter of said absorbent body in opposed directions.

3. The absorbent article of claim 2 wherein said panels (106; 406) each include a sloping edge.

4. The absorbent article of claim 1 wherein said absorbent body (102; 402) includes opposed leg cut-outs.

5. The absorbent article of claim 1 further comprising an attachment member (110; 410) coupled to said front portion (101; 401) of said absorbent body, wherein said fastener (124, 126; 424) engages said attachment member (110; 410).

6. The absorbent article of claim 5, said attachment member (110; 410) being formed from loop material.

7. The absorbent article of claim 1, said fastener (124,126; 424) coupled to said elastic member (120; 420) being formed from hook material.

8. The absorbent article of claim 1, wherein said at least one panel (106; 406) is attached to said back portion (103; 403) of said absorbent body by an adhesive bond, an ultrasonic bond, or a heat seal (108; 408).

9. The absorbent article of claim 1, wherein said at least one panel (106; 406) is continuous and extends outwardly from opposing sides of said perimeter of said absorbent body.

10. The absorbent article of claim 1, wherein said non-woven, non-elastic, breathable material is formed from a spunbond-meltblow-spunbond web.

11. A method for producing an absorbent article (100; 400), said method comprising the step of:
attaching at least one panel (106; 406) formed of a non-woven material to a back portion (103; 403) of a substantially rectangular absorbent body (102; 402) such that the at least one panel extends outwardly from a perimeter of the absorbent body;
**characterized in that** said non-woven material is non-elastic and breathable, and **in that** said method further comprises the steps of attaching an elastic member (120; 420) to the at least one panel such that the elastic member extends outwardly from the at least one panel; and
coupling a fastener (124, 126; 424) to the elastic member, wherein the fastener is configured to engage the front portion (101; 401) of the absorbent body.

12. The method of claim 11 further comprising the step of engaging the fastener (124, 126; 424) with a front portion (101; 401) of the absorbent body to form a pre-attached absorbent article.

13. The method of claim 11 or 12 further comprising the step of attaching another panel (106) formed of a non-woven, non-elastic, breathable material to the back portion (103; 403) of the absorbent body such that the panels extend outwardly from the perimeter of the absorbent body in opposing directions.

14. The method of claim 13 further comprising the step of separating the panels (106; 406) from a non-woven, non-elastic, breathable material prior to the step of attaching the panels to the absorbent body.

15. The method of claim 11 or 12 further comprising the step of coupling an attachment member (110; 410) to a front portion (101; 401) of the absorbent body.

16. The method of claim 15 further comprising the step of folding the absorbent body so that the attachment member (110; 410) is aligned with the fastener (124, 126; 424) that is coupled to the elastic member (120; 420).

17. The method of claim 16 further comprising the step of engaging the attachment member (110; 410) with the fastener (124, 126; 424) that is coupled to the elastic member (120; 420).

18. The method of claim 11 or 12 further comprising the step of positioning the at least one panel (106; 406) such that portions of the at least one panel extend outwardly from opposing sides of the perimeter of the absorbent body.

## Patentansprüche

1. Saugfähiger Artikel (100; 400), aufweisend:
einen saugfähigen Körper (102; 402) mit einem im Wesentlichen rechteckigen Umfang, einem vorderen Abschnitt (101; 401), der derart gestaltet ist, dass er gegen eine Vorderseite eines Trägers positioniert ist, und einem hinteren Abschnitt (103; 403), der derart gestaltet ist, dass er gegen eine Rückseite eines Trägers positioniert ist;
mindestens eine Bahn (106; 406), die an dem hinteren Abschnitt des saugfähigen Körpers angebracht ist und sich von dem Umfang des saugfähigen Körpers nach außen erstreckt, wobei die Bahn aus einem oder mehreren Vliesmaterialien gebildet ist;
**dadurch gekennzeichnet, dass** der saugfähige Artikel des Weiteren ein elastisches Element (120; 420) aufweist, das an der mindestens einen Bahn angebracht ist und sich von dieser nach außen erstreckt, und dass das Vliesmaterial nicht elastisch und atmungsfähig ist; und
ein Befestigungsmittel (124; 126; 424), das für einen Eingriff mit dem vorderen Abschnitt des saugfähigen Körpers an das elastische Element gekoppelt ist.

2. Saugfähiger Artikel nach Anspruch 1, wobei der saugfähige Artikel zwei Bahnen (106; 406) enthält, die an dem hinteren Abschnitt des saugfähigen Körpers angebracht sind und sich von dem Umfang des saugfähigen Körpers in entgegengesetzte Richtungen erstrecken.

3. Saugfähiger Artikel nach Anspruch 2, wobei die Bahnen (106; 406) jeweils eine schräge Kante enthalten.

4. Saugfähiger Artikel nach Anspruch 1, wobei der saugfähige Körper (102; 402) gegenüberliegende Beinausschnitte enthält.

5. Saugfähiger Artikel nach Anspruch 1, des Weiteren aufweisend ein Aufnahmeelement (110; 410), das an den vorderen Abschnitt (101; 401) des saugfähigen Körpers gekoppelt ist, wobei das Befestigungselement (124, 126; 424) mit dem Aufnahmeelement (110; 410) in Eingriff gelangt.

6. Saugfähiger Artikel nach Anspruch 5, wobei das Aufnahmeelement (110; 410) aus einem Schlingenmaterial gebildet ist.

7. Saugfähiger Artikel nach Anspruch 1, wobei das Befestigungselement (124, 126; 424), das an das elastische Element (120; 420) gekoppelt ist, aus einem Hakenmaterial gebildet ist.

8. Saugfähiger Artikel nach Anspruch 1, wobei die mindestens eine Bahn (106; 406) an dem hinteren Abschnitt (103; 403) des saugfähigen Körpers durch eine Klebebindung, eine Ultraschallbindung oder ein Heißsiegel (108; 408) angebracht ist.

9. Saugfähiger Artikel nach Anspruch 1, wobei die mindestens eine Bahn (106; 406) kontinuierlich ist und sich von gegenüberliegenden Seiten des Umfangs des saugfähigen Körpers nach außen erstreckt.

10. Saugfähiger Artikel nach Anspruch 1, wobei das nicht elastische, atmungsfähige Vliesmaterial aus einer spinngebundenen-schmelzgeblasenenspinngebundenen Stoffbahn gebildet ist.

11. Verfahren zur Herstellung eines saugfähigen Artikels (100; 400), wobei das Verfahren folgenden Schritt aufweist:
Anbringen mindestens einer Bahn (106; 406), die aus einem Vliesmaterial gebildet ist, an einem hinteren Abschnitt (103; 403) eines im Wesentlichen rechteckigen saugfähigen Körpers (102; 402), so dass sich die mindestens eine Bahn von einem Umfang des saugfähigen Körpers nach außen erstreckt;
**dadurch gekennzeichnet, dass** das Vliesmaterial nicht elastisch und atmungsfähig ist, und dass das Verfahren des Weiteren die Schritte des Anbringens eines elastischen Elements (120; 420) an der mindestens einen Bahn derart, dass sich das elastische Element von der mindestens einen Bahn nach außen erstreckt; und
des Koppelns eines Befestigungsmittels (124, 126; 424) an das elastische Element enthält, wobei das Befestigungsmittel derart gestaltet ist, dass es mit dem vorderen Abschnitt (101; 401) des saugfähigen Körpers in Eingriff gelangt.

12. Verfahren nach Anspruch 11, des Weiteren aufweisend den Schritt des In-Eingriff-Bringens des Befestigungsmittels (124, 126; 424) mit einem vorderen Abschnitt (101; 401) des saugfähigen Körpers zur Bildung eines im Voraus befestigten, saugfähigen Gegenstandes.

13. Verfahren nach Anspruch 11 oder 12, des Weiteren aufweisend den Schritt des Anbringens einer weiteren Bahn (106), die aus einem nicht elastischen, atmungsfähigen Vliesmaterial gebildet ist, an dem hinteren Abschnitt (103; 403) des saugfähigen Gegenstandes derart, dass sich die Bahnen von dem Umfang des saugfähigen Körpers in entgegengesetzte Richtungen erstrecken.

14. Verfahren nach Anspruch 13, des Weiteren aufweisend den Schritt des Trennens der Bahnen (106; 406) von einem nicht elastischen, atmungsfähigen Vliesmaterial vor dem Anbringen der Bahnen an dem saugfähigen Körper.

15. Verfahren nach Anspruch 11 oder 12, des Weiteren aufweisend den Schritt des Koppelns eines Aufnahmeelements (110; 410) an einem vorderen Abschnitt (101; 401) des saugfähigen Körpers.

16. Verfahren nach Anspruch 15, des Weiteren aufweisend den Schritt des Faltens des saugfähigen Körpers derart, dass das Aufnahmeelement (110; 410) mit dem Befestigungselement (124, 126; 424) ausgerichtet ist, das an das elastische Element (120; 420) gekoppelt ist.

17. Verfahren nach Anspruch 16, des Weiteren aufweisend den Schritt des In-Eingriff-Bringens des Aufnahmeelements (110; 410) mit dem Befestigungselement (124, 126; 424), das an das elastische Element (120; 420) gekoppelt ist.

18. Verfahren nach Anspruch 11 oder 12, des Weiteren aufweisend den Schritt des Positionierens der mindestens einen Bahn (106; 406) derart, dass sich Abschnitte der mindestens einen Bahn von gegenüberliegenden Seiten des Umfangs des saugfähigen Körpers nach außen erstrecken.

## Revendications

1. Article absorbant (100 ; 400) comprenant :
un corps absorbant (102 ; 402) ayant un périmètre sensiblement rectangulaire, une partie avant (101 ; 401) conçue pour être placée contre le devant d'un porteur, et une partie arrière (103 ; 403) conçue pour être placée contre le dos d'un porteur ;
au moins un panneau (106 ; 406) fixé à ladite partie arrière dudit corps absorbant et s'étendant vers l'extérieur depuis ledit périmètre dudit corps absorbant, ledit panneau étant formé à partir d'un ou de plusieurs matériaux non-tissés ;
**caractérisé en ce que** ledit article absorbant comprend, en outre, un élément élastique (120 ; 420) fixé audit au moins un panneau et s'étendant vers l'extérieur de celui-ci, et **en ce que** ledit matériau non-tissé est non élastique et respirant ; et
une attache (124, 126 ; 424) accouplée audit élément élastique pour engager ladite partie avant dudit corps absorbant.

2. Article absorbant selon la revendication 1, dans lequel ledit article absorbant comprend deux panneaux (106 ; 406) fixés à ladite partie arrière dudit corps absorbant et s'étendant vers l'extérieur depuis ledit périmètre dudit corps absorbant dans des directions opposées.

3. Article absorbant selon la revendication 2, dans lequel lesdits panneaux (106 ; 406) comprennent chacun un bord incliné.

4. Article absorbant selon la revendication 1, dans lequel ledit corps absorbant (102 ; 402) comprend des découpes pour jambes, opposées.

5. Article absorbant selon la revendication 1, comprenant, en outre, un élément de fixation (110 ; 410) accouplé à ladite partie avant (101 ; 401) dudit corps absorbant, ladite attache (124, 126 ; 424) s'engageant avec ledit élément de fixation (110 ; 410).

6. Article absorbant selon la revendication 5, ledit élément de fixation (110 ; 410) étant formé à partir d'un matériau à bouclettes.

7. Article absorbant selon la revendication 1, ladite attache (124, 126 ; 424) accouplée audit élément élastique (120 ; 420) étant formée à partir d'un matériau à crochets.

8. Article absorbant selon la revendication 1, dans lequel ledit au moins un panneau (106 ; 406) est fixé à ladite partie arrière (103 ; 403) dudit corps absorbant par une liaison adhésive, une liaison par ultrasons, ou un thermoscellage (108 ; 408).

9. Article absorbant selon la revendication 1, dans lequel ledit au moins un panneau (106 ; 406) est continu et s'étend vers l'extérieur depuis des côtés opposés dudit périmètre dudit corps absorbant.

10. Article absorbant selon la revendication 1, dans lequel ledit matériau non-tissé, non élastique et respirant est formé à partir d'un voile obtenu par filage/nappage-extrusion/soufflage-filage/nappage.

11. Procédé pour produire un article absorbant (100 ; 400), ledit procédé comprenant l'étape consistant à :
fixer au moins un panneau (106 ; 406) formé d'un matériau non-tissé à une partie arrière (103 ; 403) d'un corps absorbant (102 ; 402) sensiblement rectangulaire de manière que ledit au moins un panneau s'étende vers l'extérieur depuis un périmètre du corps absorbant ;
**caractérisé en ce que** ledit matériau non-tissé est non élastique et respirant, et **en ce que** ledit procédé comprend, en outre, les étapes consistant à fixer un élément élastique (120 ; 420) audit au moins un panneau de manière que l'élément élastique s'étende vers l'extérieur depuis ledit au moins un panneau ; et
accoupler une attache (124, 126 ; 424) à l'élément élastique, l'attache étant conçue pour s'engager avec la partie avant (101 ; 401) du corps absorbant.

12. Procédé selon la revendication 11, comprenant, en outre, l'étape consistant à engager l'attache (124, 126 ; 424) avec une partie avant (101 ; 401) du corps absorbant pour former un article absorbant préfixé.

13. Procédé selon la revendication 11 ou 12, comprenant l'étape consistant à fixer un autre panneau (106) formé d'un matériau non-tissé, non élastique, respirant à la partie arrière (103 ; 403) du corps absorbant de manière que les panneaux s'étendent vers l'extérieur depuis le périmètre du corps absorbant dans des directions opposées.

14. Procédé selon la revendication 13, comprenant, en outre, l'étape consistant à séparer les panneaux (106 ; 406) d'un matériau non-tissé, non élastique, respirant, avant l'étape de fixation des panneaux au corps absorbant.

15. Procédé selon la revendication 11 ou 12, comprenant, en outre, l'étape consistant à accoupler un élément de fixation (110 ; 410) à une partie avant (101 ; 401) du corps absorbant.

16. Procédé selon la revendication 15, comprenant, en outre, l'étape consistant à plier le corps absorbant de manière que l'élément de fixation (110 ; 410) soit aligné avec l'attache (124, 126 ; 424) qui est accouplée à l'élément élastique (120 ; 420).

17. Procédé selon la revendication 16, comprenant, en outre, l'étape consistant à engager l'élément de fixation (110 ; 410) avec l'attache (124, 126 ; 424) qui est accouplée à l'élément élastique (120 ; 420).

18. Procédé selon la revendication 11 ou 12, comprenant, en outre, l'étape consistant à placer ledit au moins un panneau (106 ; 406) de manière que des parties dudit au moins un panneau s'étendent vers l'extérieur depuis des côtés opposés du périmètre du corps absorbant.
